# EUROPEAN PATENT APPLICATION

(11) **EP 3 006 054 A1**
(43) Date of publication of application: **13.04.2016**
(21) Application number: 14807133.5
(22) Date of filing: 02.06.2014
(51) Int. Cl.: A61L 27/00

(54) **MATERIAL FOR REGENERATION OF PERIODONTAL TISSUE**

(30) Priority: 06.06.2013 JP 2013119441
(71) Applicant: Nihon University, Tokyo 102-8275 (JP)
(72) Inventor: HONDA, Masaki, Tokyo 102-8275 (JP); AKITA, Daisuke, Tokyo 102-8275 (JP); KANO, Koichiro, Tokyo 102-8275 (JP); MATSUMOTO, Taro, Tokyo 102-8275 (JP); KANEKO, Tadashi, Tokyo 174-8585 (JP); YAMANAKA, Katsuyuki, Tokyo 174-8585 (JP); SAKAI, Yuuhiro, Tokyo 174-8585 (JP)
(74) Representative: Høiberg A/S
(86) International application number: PCT/JP2014/064633
(87) International publication number: WO 2014/196503

(57) **Abstract**

It is a problem of the present invention to provide a convenient and safe periodontal tissue regeneration material and provide a method of regenerating a periodontal tissue. The present invention provides a periodontal tissue regeneration material comprising dedifferentiated fat cells (DFAT) as the convenient and safe periodontal tissue regeneration material. The present invention provides a method of regenerating a periodontal tissue with the periodontal tissue regeneration material.

## Description

### TECHNICAL FIELD

The present invention relates to a periodontal tissue regeneration material comprising dedifferentiated fat cells (DFAT (hereinafter sometimes referred to as DFAT)).

The present invention also relates to a method of regenerating a periodontal tissue with the periodontal tissue regeneration material.

### BACKGROUND ART

A periodontal tissue is destroyed by occurrence of inflammation in the periodontal tissue due to plaque bacteria and metabolite thereof. The destroyed periodontal tissue cannot be regenerated by a method of treatment of removing a cause such as plaque and, therefore, various periodontal tissue regeneration techniques are recently disclosed.

For example, Patent Document 1 discloses a tissue or organ regeneration material acquired by culturing stem cells on a cell support body. It is confirmed that the material comprising mesenchymal stem cells exhibits favorable osteogenic ability in transplantation to a bone defect site of a canine lower jawbone region.

In Patent Document 2, differentiation is confirmed from the SSEA-4 positive mesenchymal stem cells isolated from a tooth or periodontal tissue to adipocytes, osteoblasts, chondrocytes, etc. In this document, it is described that a tooth or periodontal tissue differentiated from the SSEA-4 positive mesenchymal stem cells is transplanted to a periodontal tissue to regenerate the periodontal tissue. Patent Document 3 relates to a method of culturing milk-tooth dental pulp mesenchymal stem cells and permanent-tooth dental pulp mesenchymal stem cells and it is described that cells cultured with this method are used for regeneration of periodontal tissues etc.

In another periodontal tissue regeneration technique disclosed in Patent document 4, a proliferation promoter and a differentiation promoter for cells comprising BMP-2, heparan sulfate, heparin, etc. as active ingredients are used as a formation promotion and regeneration promoter for periodontal tissues.

Regeneration of periodontal tissues by an adipose tissue-originated interstitial cell group is also under study (Non-Patent Document 1).

Although these techniques are useful for regeneration of periodontal tissues, for example, the mesenchymal stem cells as described in Patent Documents 1 to 3 and the isolation of the mesenchymal stem cells from the periodontal tissues require tooth extraction from a human etc. providing materials and have problems of significant invasion/pain etc., and these techniques are therefore not considered as practical methods. Although the interstitial fat cell culture of Non-Patent Document 1 enables convenient collection of a large amount of cells, differences may be generated in transplantation results depending on age and case because various cells are included.

Therefore, it is desired to provide a more convenient and safe periodontal tissue regeneration material and provide a method of regenerating a periodontal tissue.

Dedifferentiated fat cells (DFAT) are known as cells different from the stem cells as disclosed in these Patent Documents and having the same differentiation potency as the stem cells. The DFAT is cells spontaneously starting dedifferentiation to acquire multipotency when mature fat cells making up a fat tissue are isolated and subjected to ceiling culture, and is known as being capable of differentiation to myoblasts and chondrocytes (Patent Document 5)

However, the case of using the DFAT for the regeneration of a periodontal tissue has not been known. A study is reported for a possibility of regeneration of periodontal tissues by creating a bone defect portion in a rat maxilla molar part and transplanting the DFAT along with a carrier (scaffold) made of atelocollagen sponge (Non-Patent Document 2). However, in this report, the regeneration of periodontal tissues after transplantation was recognized in both a group in which both the DFAT and the carrier are transplanted and a group in which only the carrier is transplanted, and a significant difference there between is not clarified. Therefore, it cannot be said that the regeneration ability of the DFAT itself is confirmed. Moreover, the report does not include description on whether the carrier is effective.

Although this document describes that GFP positive cells are recognized in a new bone surface, a new periodontal membrane and a connective tissue, it is not clear whether a tissue is regenerated by DFAT cells, and only the possibility of involvement of the DFAT with the periodontal tissue regeneration is described.

### CITATION LIST

### PATENT LITERATURE

Patent Document 1: Japanese Laid-Open Patent Publication No. 2005-278910
Patent Document 2: WO 2010/016492
Patent Document 3: Japanese Laid-Open Patent Publication No. 2010-268715
Patent Document 4: Japanese Laid-Open Patent Publication No. 2008-74732
Patent Document 5: WO2004/111211

### NON PATENT LITERATURE

Non-Patent Document 1: Japan Prosthodontic Society (Public Interest Incorporated Association), Program and Abstracts, The 122nd Scientific Meeting of the Japan Prosthodontic Society, the 80th Anniversary, p 132
Non-Patent Document 2: Japanese Society of Conservative Dentistry (Non-Profit Organization), Program and Abstracts (Web), The 133rd Meeting of the Japanese Society of Conservative Dentistry, 2010, p181 "A Study of Periodontal Tissue Regenerative by Using Rat Dedifferentiated Fat Cell"

### SUMMARY OF INVENTION

### TECHNICAL PROBLEM

It is a problem of the present invention to provide a convenient and safe periodontal tissue regeneration material and provide a method of regenerating a periodontal tissue. Particularly, it is a problem of the present invention to provide a periodontal tissue regeneration method with high practical value using DFAT.

### SOLUTION TO PROBLEM

As a result of intensive studies for solving the problems, the present inventors found that dedifferentiated fat cells (DFAT) effectively act in regeneration of periodontal tissues, thereby completing the provision of a periodontal tissue regeneration material comprising the cells. The periodontal tissue regeneration material enables the provision of a method of regenerating a periodontal tissue.

The dedifferentiated fat cells comprised in the periodontal tissue regeneration material of the present invention are cells acquired with high purity in a large amount through ceiling culture from a fat tissue conveniently available to dentists etc., and therefore are likely to produce more highly biologically safe and more stable therapeutic effect as compared to a conventional periodontal tissue regeneration therapy using a interstitial cell group made up of various cells.

Thus, the present invention is as follows.
[1] A periodontal tissue regeneration material comprising dedifferentiated fat cells.
[2] The periodontal tissue regeneration material according to [1], further comprising a carrier.
[3] The periodontal tissue regeneration material according to [2], wherein the carrier is a carrier mainly comprising poly(lactic-co-glycolic acid) (PLGA).
[4] The periodontal tissue regeneration material according to [2], wherein the carrier is a carrier mainly comprising poly(lactic-co-glycolic acid) (PLGA) and having a porosity of 60 % or more and 95 % or less.
[5] The periodontal tissue regeneration material according to any one of [2] to [4], wherein the carrier is a block-shaped carrier acquired by molding granular poly(lactic-co-glycolic acid) (PLGA).
[6] The periodontal tissue regeneration material according to any one of [1] to [5], wherein the periodontal tissue regeneration material is used in combination with a tissue regeneration absorbent membrane.
[7] A periodontal tissue regeneration kit comprising the periodontal tissue regeneration material according to any one of [1] to [6] and a tissue regeneration absorbent membrane.
[8] The periodontal tissue regeneration kit according to [7], wherein the tissue regeneration absorbent membrane is a poly(lactic-co-glycolic acid) membrane or a collagen membrane.
[9] A method of regenerating a periodontal tissue comprising a step of transplanting the periodontal tissue regeneration material according to any one of [1] to [6] to a periodontal tissue defect portion.
[10] The method of regenerating a periodontal tissue according to [9], further comprising a step of covering a transplantation portion with a tissue regeneration absorbent membrane.

### ADVANTAGEOUS EFFECTS OF INVENTION

The provision of the periodontal tissue regeneration material of the present invention and the provision of the method of regenerating a periodontal tissue with the periodontal tissue regeneration material enable the provision of safe, convenient, and useful therapeutic agent, therapy, etc. against disease causing a periodontal tissue defect such as periodontal disease.

### BRIEF DESCRIPTION OF DRAWINGS

[Fig. 1] Fig. 1 is a diagram of observed dedifferentiated fat cells (fibroblast-like cells) (Example 1).
[Fig. 2] Fig. 2 is a diagram of a PLGA carrier after pretreatment for seeding of the dedifferentiated fat cells (Example 1).
[Fig. 3] Fig. 3 is a diagram of a defect site in a periodontal tissue defect model (Example 2).
[Fig. 4] Fig. 4 is a diagram of hard tissue amounts in transplantation groups after transplantation (Example 2).
[Fig. 5] Fig. 5 is a diagram of confirmation of regeneration of the periodontal tissues in the transplantation groups after transplantation (Example 2).
[Fig. 6] Fig. 6 is a diagram of confirmed regeneration of the cement in the transplantation groups after transplantation (Example 2).
[Fig. 7] Fig. 7 is a diagram of confirmed regeneration of the cement in the transplantation groups after transplantation (Example 2).
[Fig. 8] Fig. 8 is a diagram of a regeneration rate of the cement in the transplantation groups after transplantation (Example 2).

### DESCRIPTION OF EMBODIMENTS

A "periodontal tissue regeneration material" of the present invention refers to a material for restoring a part subjected to destruction, defect, etc. so that the part may function as a periodontal tissue in the case of destruction, defect, etc. of the periodontal tissues made up of four tissues, i.e., the soft tissues of the gum and the periodontal membrane and the hard tissues of the cement and the alveolar bone, due to a biological action such as periodontal disease, a physical action, or a mechanical action, for example.

The "periodontal tissue regeneration material" of the present invention is a material that may regenerate at least one or more tissues, for example, the cement or the periodontal membrane out of the tissues making up the periodontal tissues, and is preferably a material that may regenerate all the tissues making up the periodontal tissues.

The "periodontal tissue regeneration material" of the present invention may be a periodontal tissue regeneration material comprising dedifferentiated fat cells. The "dedifferentiated fat cells" refer to undifferentiated fibroblast-like cells acquired through ceiling culture etc. of mature fat cells acquired from the fat tissues of animals such as humans, pigs, dogs, and birds due to dedifferentiation of the fat cells. Such "dedifferentiated fat cells" of the present invention have multipotency that may enable differentiation to cells having functions other than that of the fat cells, for example, osteoblasts, myoblasts, or nerve cells.

The present invention enables the use of the "dedifferentiated fat cells" acquired by any conventionally known methods and enables the use of the cells after long-term passage for several generations or several tens of generations of the primary dedifferentiated fat cells acquired in this way.

The "periodontal tissue regeneration material" of the present invention preferably further comprises a "carrier" in addition to the dedifferentiated fat cells. The "carrier" of the present invention preferably acts as a scaffold for the dedifferentiated fat cells reconstructing the periodontal tissues or a place for proliferation of the dedifferentiated fat cells, and may be any conventionally known carrier as long as the carrier is safe for animals etc., in which the "periodontal tissue regeneration material" of the present invention is transplanted.

Preferably, such a "carrier" of the present invention is, for example, a carrier made of an absorbent material, preferably a carrier mainly comprising poly(lactic-co-glycolic acid) (PLGA). This carrier is preferably a block-shaped PLGA carrier mainly comprising poly(lactic-co-glycolic acid) (PLGA) and having a porosity of 60 % or more and 95 % or less and is particularly preferably a block-shaped PLGA carrier having a porosity of 80 %.

A carrier having a porosity less than 60 % or greater than 95 % may also be used as the "carrier" of the present invention; however, a porosity less than 60 % leads to poor formability making a communication property and a pore size of pores smaller although the strength is made higher, and therefore makes it difficult to uniformly seed the dedifferentiated fat cells inside the carrier. A porosity greater than 95 % makes a communication property and a pore size of pores larger and improve the seeding property for the dedifferentiated fat cells; however, since the dedifferentiated fat cells are hardly retained inside the carrier and the strength of the carrier is reduced, it is difficult to maintain a shape during culture or after transplantation.

The pore size may be 100 µm to 500 µm, more preferably 150 to 400 µm.

In the regeneration of the periodontal tissues of the present invention, it is important for improvement in tissue regeneration efficiency that both the pore size and the porosity are within proper ranges and, preferably, the pore size is 100 µm to 500 µm while the porosity is 60 % or more and 95 % or less, and more preferable ranges are the pore size of 150 to 400 µm and the porosity of 60 % or more and 90 % or less.

Specifically, for example, the carrier may have the porosity of 80 % and the pore size of about 180 µm or the porosity of 90 % and the pore size of about 350 µm.

Although the carrier of the present invention may be a spongy copolymer made of lactic acid and glycolic acid prepared by a freeze-drying method or a leaching method, the carries prepared by these methods are difficult to maintain a shape during a culture period or after transplantation because of low strength and makes it difficult to uniformly seeding the dedifferentiated fat cells inside the carrier because pores are highly independent. Therefore, it is preferable to use the carrier mainly comprising poly(lactic-co-glycolic acid) (PLGA) having higher strength.

The carrier mainly comprising poly(lactic-co-glycolic acid) (PLGA) of the present invention is acquired by molding of a granular material and therefore have high strength and the communication property of pores, which can compensate for shortcomings of the spongy carrier. The use of the block-shaped PLGA carrier of the present invention is also advantageous in that a place allowing cell growth can be maintained wider at the time of transplantation so as to maintain a place for tissue formation as compared to the spongy carrier.

The shape of the block may be any shape matching a shape of a defect portion subjected to transplantation and examples of the cross sectional shape thereof include a circle, a triangle, a quadrangle, and other polygonal shapes, including a square, a rectangle, a trapezoid, a rhombus, etc. as a quadrangle.

The "periodontal tissue regeneration material" of the present invention is preferably used with the dedifferentiated fat cells seeded to the carrier and engrafted in the carrier. The dedifferentiated fat cells may be engrafted to any location of the carrier such as on the carrier and inside the carrier and are particularly preferably engrafted inside the carrier.

Although the engraftment of the dedifferentiated fat cells to the carrier may be achieved by using any conventionally known methods including, for example, a method in which the dedifferentiated fat cells are seeded to the carrier subjected to pretreatment etc., and allowed to stand still for a certain time for engraftment.

The number of the dedifferentiated fat cells engrafted to the carrier in the "periodontal tissue regeneration material" of the present invention may be any number enabling the regeneration of the periodontal tissue and may be adjusted in accordance with the size of the carrier etc. The size of the carrier used can be adjusted in accordance with a location of the periodontal tissue desirably regenerated by transplanting the "periodontal tissue regeneration material" of the present invention, such as a size of the defect portion of the periodontal tissue.

The "periodontal tissue regeneration material" of the present invention is more preferably used in combination with a tissue regeneration absorbent membrane. By using in combination with the "tissue regeneration absorbent membrane," the "periodontal tissue regeneration material" of the present invention can be blocked from tissues outside the periodontal tissues including the periosteum and the fascia associated with the masseter to avoid the intrusion of cells inhibiting the regeneration of the periodontal tissues, thereby enabling the enhancement of the regeneration promoting abilities of the dedifferentiated fat cells and the carrier.

The "tissue regeneration absorbent membrane" preferably prevent the separation of the dedifferentiated fat cells and the carrier containing the dedifferentiated fat cells from the location of transplantation and the intrusion etc. of cells inhibiting the regeneration of the periodontal tissues into the cattier and may be any conventionally known membrane safe for animals etc., in which the "periodontal tissue regeneration material" of the present invention is transplanted.

Such a "tissue regeneration absorbent membrane" may be any conventionally known membrane and may be a commercially available membrane. Examples of such a "tissue regeneration absorbent membrane" include, for example, Koken Tissue Guide (collagen membrane; Koken Co., Ltd.), Biomend (collagen membrane; Hakuho Corporation), or GC Membrane (poly(lactic-co-glycolic acid) membrane; GC Corporation). The examples may also include Vicryl Mesh (polyglactin; J&J), INION GTR (registered trademark) (PLLA; INION), or BIO-GUIDE (registered trademark) (collagen; GEISTLICH-PHARMA) commercially available in foreign countries.

A "method of regenerating a periodontal tissue" of the present invention may be a method comprising a step of transplanting the "periodontal tissue regeneration material" of the present invention and thereby enabling the regeneration of the periodontal tissues. The method may be a method including this step and may include another method useful for the regeneration of the periodontal tissues.

The present invention will hereinafter specifically be described with reference to examples; however, the present invention is obviously not limited thereto.

### Examples

### [Example 1]

### Preparation of Periodontal Tissue Regeneration Material

A periodontal tissue regeneration material was prepared through the following steps of 1) and 2).

### 1) Preparation of Dedifferentiated Fat Cells (DFAT)

About 1 g of subcutaneous fat tissues collected from the groin of 8-week-old male F344 rats (CLEA Japan) was washed and then enzyme-treated with a 0.1 % collagenase (SIGMA) solution. After removing excess tissues by a 100 µm cell strainer (BD Falcon), the tissues were centrifuged at 135 G for 3 minutes to collect a mature fat cell fraction floating in an upper portion of a centrifuge tube.

After a collected mature fat cell group was washed thrice with Dulbecco's Modified Eagle Medium (DMEM medium; SIGMA), 5×10⁴ cells were transferred to a 25 cm² flask filled with a DMEM medium to which 20 % FETAL BOVINE SERUM (FBS; Nichirei Biosciences. INC) and 1 % Pen Strep (GIBCO) were added, and the mature fat cell group adhered to a ceiling portion of the inverted flask in the medium (ceiling culture).

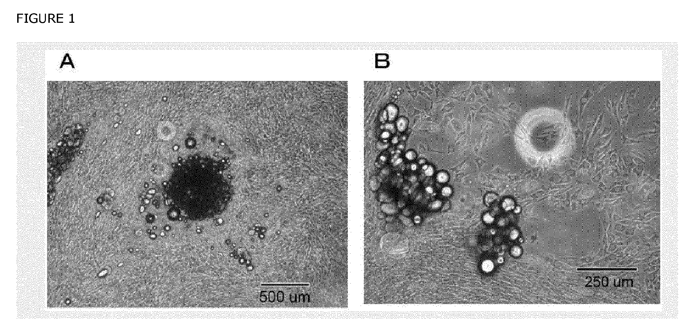

By continuously culturing the cells adhering to the ceiling portion in the same culture solution for 7 days, the mature fat cells dedifferentiated after seven days, and fibroblast-like cells exhibiting a uniform form were observed (Fig. 1, A and B).

After the fibroblast-like cells were confirmed and the flask was returned to the normal orientation, the medium was replaced to continue the culture and the fibroblast-like cells continuously proliferated. These fibroblast-like cells were used as the dedifferentiated fat cells (DFAT). The flask was inverted during the ceiling culture so that the fibroblast-like cells can be cultured in the normal orientation.

The dedifferentiated fat cells acquired from the mature fat cells through dedifferentiation were subjected to subculture operations in the usual manner using a trypsin-EDTA solution to acquire the necessary number of cells. These dedifferentiated fat cells were examined in terms of differentiation potency to osteoblasts and adipocytes and were confirmed as the dedifferentiated fat cells having the multipotency.

Although cells of the third generation were used for the regeneration of the periodontal tissues in this preparation of the periodontal tissue regeneration material, any dedifferentiated fat cells acquired through dedifferentiation from the mature fat cells can be used for the preparation of the periodontal tissue regeneration material regardless of whether the cells are of the first generation or those after long-term passage of several tens of generations.

### 2) Seeding of Dedifferentiated Fat Cells to Carrier

### (1) Preparation of Carrier

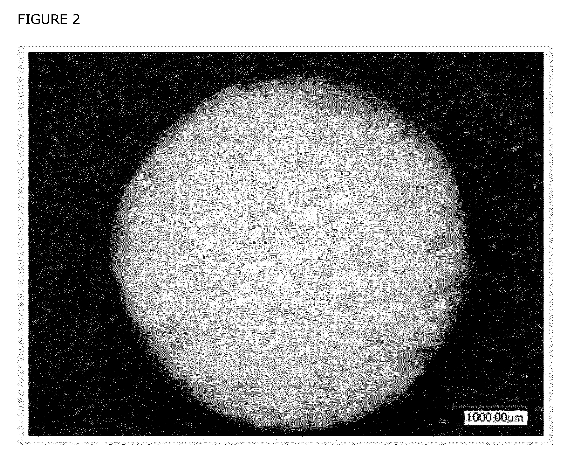

A block-shaped PLGA carrier mainly comprising poly(lactic-co-glycolic acid) (PLGA) and having the porosity of 80 % (size: 2 mm in length, 3 mm in width, 1 mm in thickness, 180 µm in pore size; manufactured by GC Corporation) was used. The carrier was subjected to a degassing treatment (Fig. 2) with 70 % ethanol and pretreated for seeding of the dedifferentiated fat cells.

### (2) Seeding of Dedifferentiated Fat Cells

After the PLGA carrier prepared in (1) is immersed in the DMEM medium for 24 hours, a cell suspension (1.0×10⁶ cells/200µl) containing the dedifferentiated fat cells prepared in 1) was added to the upper surface of the carrier to seed the dedifferentiated fat cells. The cells were allowed to stand still in the DMEM medium at 37 °C under 5 % CO₂ for 6 hours and the number of leaked cells was measured after standing to confirm that the dedifferentiated fat cells were engrafted inside the carrier. The material prepared in this way was used as the periodontal tissue regeneration material.

### [Example 2]

### Regeneration of Periodontal Tissues

The periodontal tissue regeneration material prepared in Example 1 was used for regenerating the periodontal tissues.

### 1. Preparation of Periodontal Tissue Defect Model

A periodontal tissue defect model was prepared in accordance with the method of King et al. (King GN et al., J Dent Res 1997; 76; 1460e70).

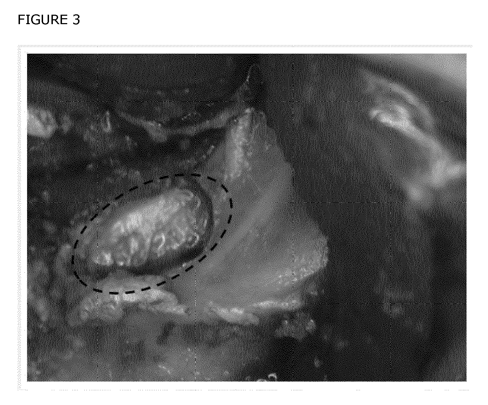

In particular, 8-week-old male F344 rats were intraperitoneally anesthetized with somnopentyl (kyoritsu Seiyaku Corporation) and subjected to hair removal and incision in the skin from the left mouth angle to the angle of the mandible to cut the masseter. The cut masseter was then reversed and the buccal side of the first molar distal root in the periphery of the exposed lower jawbone was mechanically damaged until the dentin is exposed with a dental inverted bur (Joda) under water injection. In this way, the periodontal tissue defect model having a defect site of 2 mm in length × 3 mm in width × 1 mm in depth (Fig. 3) was prepared.

### 2. Regeneration of Periodontal Tissues by Periodontal Tissue Regeneration Material

After washing the defect site of the periodontal tissue defect model prepared in 1, the defect portion was filled with the periodontal tissue regeneration material comprising the dedifferentiated fat cells prepared in Example 1 (filled only with the carrier in a control group) and then covered with a GC membrane (poly(lactic-co-glycolic acid) membrane; 7 mm long×8 mm wide; GC Corporation) for transplantation. After the transplantation, the masseter and the skin were returned to the original position and sutured. A transplantation period was 5 weeks.

A group of the periodontal tissue defect models with the transplanted periodontal tissue regeneration material was defined as a periodontal tissue regeneration material transplantation group (n=3) and, by way of comparison, a carrier transplantation group (n=3) was prepared by washing the defect site of the periodontal tissue defect model and filling the defect site with the PLGA carrier pretreated in (1) and a GC membrane (7 mm long×8 mm wide) for transplantation, followed by saturation before 5 weeks of the transplantation period.

Each of the transplantation locations (defect site peripheral portions) of the periodontal tissue regeneration material transplantation group and the carrier transplantation group was photographed every 7 days during 5 weeks of the transplantation period by an X-ray CT system (R_mCT; Rigaku) under the conditions of 90 kV, 100 mA, 20× photographing magnification (voxel size: 30×30×30 µm), and 17 seconds.

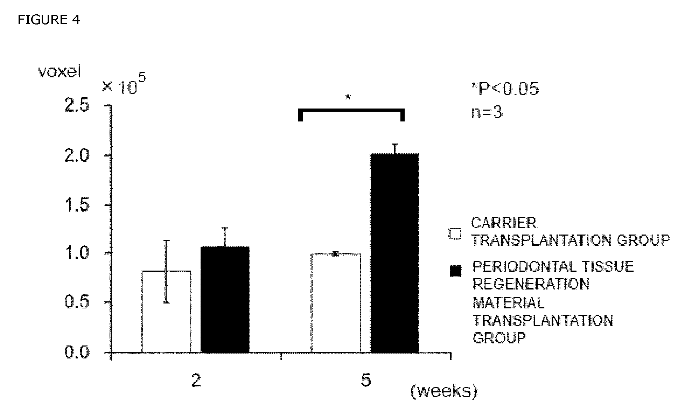

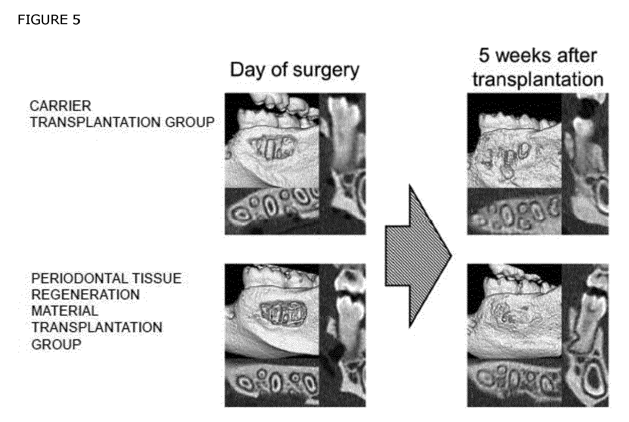

CT image processing was executed based on acquired projection data by the integrated image processing software I-view-3DX Ver. 1.82 (MORITA). A µCT image was analyzed by 3-by-4 Viewer Ver. 2.4 ((Kitasenjyu Radist Dental Clinic, I-View Image Center) to quantitatively evaluate a hard tissue amount in the prepared defect portion. Bonferroni-corrected Mann-Whitney Test was used for the significance test. As a result, it was confirmed that the hard tissue amount in the defect portion was significantly increased in the periodontal tissue regeneration material transplantation group as compared to the carrier transplantation group (Fig. 4) and the presence of a periodontal-membrane-like void was recognized between the cement and the alveolar bone (Fig. 5).

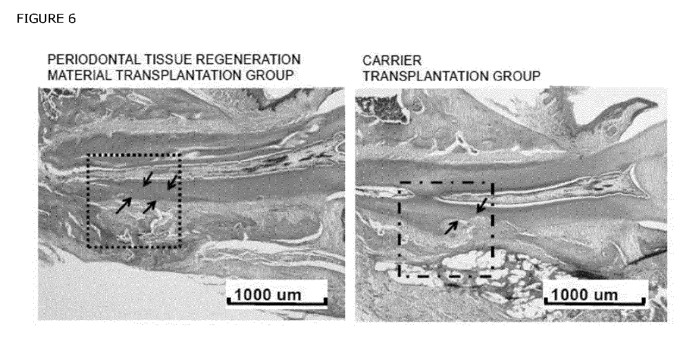

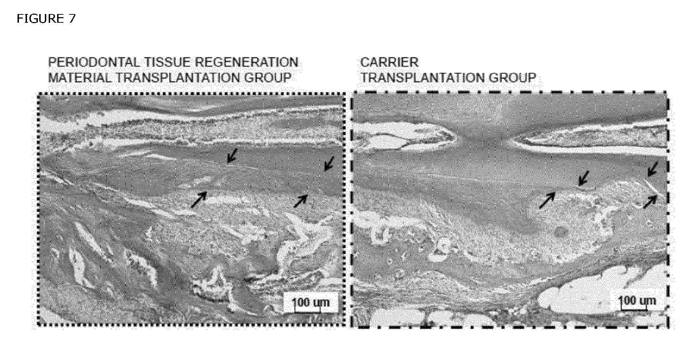

After the end of the transplantation period, the lower jawbone including the transplantation location (defect site peripheral portion) was taken out and fixed in 10 % neutral buffer formalin solution. Subsequently, the lower jawbone was subjected to a decalcification operation with an EDTA solution for 4 weeks and was dehydrated, cleared, and infiltrated for paraffin embedding in the usual manner for histological analysis. The lower jawbone was then sectioned by 7 µm and stained with hematoxylin and eosin for evaluation. As a result, the cement (portions between arrows in Figs. 6 and 7) and the alveolar bone-like and periodontal membrane-like tissues were recognized in the first molar mesial root in both the periodontal tissue regeneration material transplantation group and the carrier transplantation group.

A level of regeneration of the cement was evaluated by measuring a width of the regenerated cement. When the width was measured at a plurality of locations randomly selected from the prepared defect site peripheral portion in each of the groups, it was confirmed that the periodontal tissue regeneration material transplantation group was associated with a significantly wider width of the cement and a higher level of regeneration of the cement as compared to the carrier transplantation group.

A cement regeneration rate in each of the periodontal tissue defect models was acquired by calculating a ratio of the width of the regenerated cement in the defect portion (a ratio of the width of the cement on the buccal side with the created defect to the width of the cement on the lingual side without the defect).

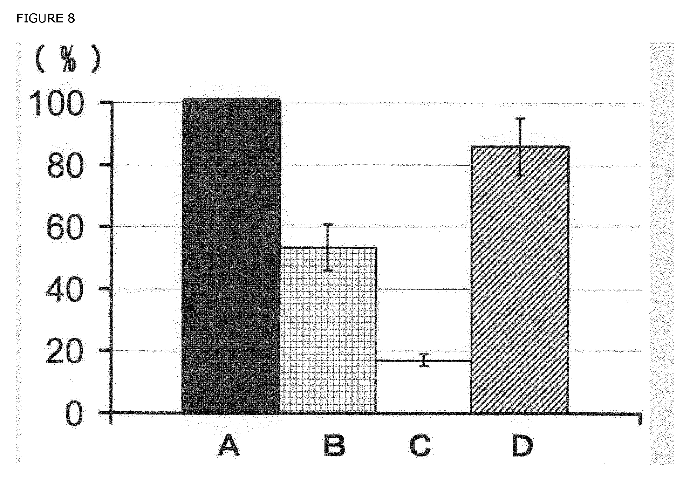

Comparing the calculated ratio with the case of the 100 % width (Fig. 8, A)of the cement of the tooth root center portion of the healthy first molar tooth maintained without a buccal peripheral defect, the regeneration rate of the cement was low in the carrier transplantation group (Fig. 8, C) and the regeneration rate of the cement was not sufficient in the group without transplantation of the carrier and the periodontal tissue regeneration material (Fig. 8, B). On the other hand, the periodontal tissue regeneration material transplantation group (Fig. 8, D) achieved 80 % of the width of the cement of the healthy periodontal tissues and it was confirmed that the regeneration rate of the cement was extremely high.

Observing the regenerated periodontal membrane, the running of the periodontal membrane fibers were observed in the arrangement orthogonal to the tooth root. It was also recognized that the fibers of the periodontal membrane were embedded in the cement. This matched the running of the fibers of the periodontal membrane found in a healthy tooth maintained without a defect, indicating that the fibers are the same as the Sharpey's fibers recognized in the healthy cement.

Thus, since the periodontal tissue regeneration material of the present invention enables the regeneration of the cement and the regeneration of a fiber group having the function of the periodontal membrane, it is confirmed that the periodontal tissue regeneration material of the present invention is useful for the regeneration of the periodontal tissues.

### INDUSTRIAL APPLICABILITY

The periodontal tissue regeneration material comprising DFAT of the present invention can achieve the regeneration of the periodontal tissues and can provide a safe, convenient, and useful therapeutic agent, therapy, etc. against disease causing a periodontal tissue defect such as periodontal disease.

## Claims

1. A periodontal tissue regeneration material comprising dedifferentiated fat cells.

2. The periodontal tissue regeneration material according to claim 1, further comprising a carrier.

3. The periodontal tissue regeneration material according to claim 2, wherein the carrier is a carrier mainly comprising poly(lactic-co-glycolic acid) (PLGA).

4. The periodontal tissue regeneration material according to claim 2, wherein the carrier is a carrier mainly comprising poly(lactic-co-glycolic acid) (PLGA) and having a porosity of 60 % or more and 95 % or less.

5. The periodontal tissue regeneration material according to any one of claims 2 to 4, wherein the carrier is a block-shaped carrier acquired by molding granular poly(lactic-co-glycolic acid) (PLGA).

6. The periodontal tissue regeneration material according to any one of claims 1 to 5, wherein the periodontal tissue regeneration material is used in combination with a tissue regeneration absorbent membrane.

7. A periodontal tissue regeneration kit comprising the periodontal tissue regeneration material according to any one of claims 1 to 6 and a tissue regeneration absorbent membrane.

8. The periodontal tissue regeneration kit according to claim 7, wherein the tissue regeneration absorbent membrane is a poly(lactic-co-glycolic acid) membrane or a collagen membrane.

9. A method of regenerating a periodontal tissue comprising a step of transplanting the periodontal tissue regeneration material according to any one of claims 1 to 6 to a periodontal tissue defect portion.

10. The method of regenerating a periodontal tissue according to claim 9, further comprising a step of covering a transplantation portion with a tissue regeneration absorbent membrane.
